# FASCICULE DE BREVET EUROPEEN

(11) **EP 2 375 971 B1**
(45) Date de publication et mention de la délivrance du brevet: **09.04.2014**
(21) Numéro de dépôt: 09801740.3
(22) Date de dépôt: 03.12.2009
(51) Int. Cl.: A61B 5/022, A61B 5/0225

(54) **MESURE DE LA PRESSION SYSTOLIQUE**
MESSUNG DES SYSTOLISCHEN DRUCKS
MEASUREMENT OF SYSTOLIC PRESSURE

(30) Priorité: 05.12.2008 FR 0858291
(43) Date de publication de la demande: 19.10.2011
(73) Titulaire: Atys Sarl, 69510 Soucieu En Jarrest (FR)
(72) Inventeur: PARZY, Denis, F-69370 Saint Didier Au Mont D'or (FR); GUIBERT, Benoît, F- 38200 Seyssuel (FR)
(74) Mandataire: Thibault, Jean-Marc
(86) Numéro de dépôt international: PCT/FR2009/052407
(87) Numéro de publication internationale: WO 2010/063976

(56) Documents cités:
- US-A1- 2005 148 885
- US-A1- 2008 077 024
- US-B1- 6 400 971

## Description

La présente invention concerne le domaine technique général des appareils non invasifs de mesure de la pression sanguine.

Plus particulièrement, la présente invention concerne le domaine des appareils de mesure de la pression sanguine systolique au doigt, et en particulier le doigt du pied.

Le brevet EP 1 217 941 décrit un dispositif optique servant à mesurer différents paramètres sanguin de manière non invasive et au moyen d'un doigtier. Le doigtier comprend un premier élément, adapté pour être maintenu sur un doigt et équipé d'une unité de mesure, et un second élément placé en amont du premier élément et équipé d'un organe d'occlusion. L'organe d'occlusion applique antérieurement à la mesure une pression supérieure à la pression sanguine systolique du patient, de manière à figer le volume sanguin dans le doigt. L'unité de mesure détermine alors en continu l'absorbance du doigt selon différentes longueurs d'ondes.

La pression sanguine systolique peut être déterminée, avec ce type de dispositif, en relâchant progressivement la pression appliquée par l'organe d'occlusion jusqu'à ce que l'unité de mesure détecte une variation de l'absorbance dans le doigt, caractéristique du retour de la pulsatilité artérielle.

Cependant, lorsque la pression sanguine systolique du patient est faible, par exemple inférieure à 30 mmHg, le flux sanguin artériel est peu pulsatile. De ce fait, la détection du retour de la pulsatilité artérielle est imprécise, et il en est de même pour la mesure de la pression sanguine systolique. Ainsi, ce type de dispositif n'est pas adapté pour la mesure de pressions sanguines systoliques faibles.

De même, la demande de brevet US 2005/148885 décrit un dispositif de mesure non invasive en quasi-continu de la pression artérielle d'une personne. Ce dispositif comporte une manchette gonflable pourvue d'un capteur photo-pléthysmographique mesurant la pression sanguine du doigt. La manchette est pilotée en gonflage/dégonflage pour maintenir le capteur appliqué sur le doigt. Un tel dispositif de mesure utilise pendant le gonflage et le dégonflage de la manchette, l'information de la composante variable du capteur pour rechercher et suivre la pression moyenne sanguine. Ce dispositif n'utilise pas pendant le dégonflage, l'information du signal du capteur pour déterminer précisément la valeur de la pression systolique

Un objet de l'invention vise à remédier aux inconvénients de l'état de la technique en permettant une mesure précise des pressions sanguines systoliques faibles.

Un autre objet de l'invention vise à proposer un procédé non invasif de mesure permettant une mesure précise des pressions sanguines systoliques faibles au doigt, et en particulier à l'orteil.

A cette fin, un procédé non invasif de mesure de la pression sanguine systolique selon l'invention comporte les étapes suivantes :
- appliquer et maintenir une pression de vidange astringente sur une première zone située à proximité de la partie distale du doigt,
- appliquer et maintenir une pression d'occlusion astringente et supérieure à la pression sanguine systolique du doigt sur une deuxième zone du doigt située en amont de la première zone,
- relâcher la pression de vidange,
- relâcher la pression d'occlusion de manière contrôlée et dans le même temps, acquérir au moins la pression d'occlusion et au moins le volume sanguin du doigt au niveau de la première zone en fonction du temps,
- calculer la pression sanguine systolique correspondant à la pression d'occlusion au moment du retour sanguin dans le doigt caractérisé par une variation sensiblement positive du volume sanguin du doigt au niveau de la première zone.

Selon une caractéristique préférée de réalisation, la pression de vidange est supérieure à la pression sanguine systolique du doigt.

De manière avantageuse, le fait de vider le doigt avant la mesure permet d'augmenter l'apport sanguin lors du retour sanguin en superposant les apports de la circulation artérielle, de la micro-circulation et de la circulation non pulsatile dans le remplissage, ce qui facilite sa mise en évidence.

De plus, un procédé de mesure selon l'invention peut présenter en outre au moins l'une des caractéristiques additionnelles suivantes :
- les pressions de vidange et d'occlusion sont appliquées et relâchées automatiquement,
- la pression de vidange est appliquée et relâchée manuellement tandis que la pression d'occlusion est appliquée et relâchée automatiquement,
- le procédé comporte une phase d'attente d'une durée comprise entre 0 et 5s et préférentiellement de l'ordre de 3s, située après le relâchement de la pression de vidange,
- l'étape de calcul de la pression sanguine systolique consiste à extraire une composante pulsatile représentant la circulation artérielle pulsatile dans le doigt, à partir de la variation du volume sanguin du doigt au niveau de la première zone en fonction du temps, puis à calculer la pression sanguine systolique par détermination de la pression d'occlusion au moment où la composante pulsatile du signal réapparaît,
- l'étape de calcul de la pression sanguine systolique consiste à extraire une composante non pulsatile représentant le remplissage du doigt résultant de la circulation artérielle pulsatile, de la microcirculation et/ou de la circulation artérielle non pulsatile, à partir de la variation du volume sanguin du doigt au niveau de la première zone en fonction du temps, puis à calculer la pression sanguine systolique en déterminant la pression d'occlusion au moment où la composante non pulsatile varie sensiblement positivement,
- le procédé consiste à ajuster le temps nécessaire au relâchement de la pression d'occlusion pour qu'il se déroule sur un nombre fixe de cycles cardiaques.

Un autre objet de l'invention vise à proposer un dispositif de mesure permettant une mesure précise des pressions sanguines systoliques faibles au doigt et en particulier de l'orteil.

A cette fin, un dispositif de mesure non invasif de la pression sanguine systolique d'un doigt comprenant une partie distale comporte, selon l'invention définie dans la revendication 9, un premier élément comprenant un capteur, apte à mesurer le volume sanguin du doigt au niveau d'une première zone située à proximité de la partie distale du doigt, et un organe de vidange, apte à appliquer ou à transmettre une pression de vidange sur la première zone du doigt, un deuxième élément comprenant un organe d'occlusion artériel, apte à appliquer et/ou à maintenir une pression d'occlusion sur une deuxième zone du doigt situé en amont de la première zone, et un dispositif de gonflage et de dégonflage asservi commandé par une électronique de commande et assurant le gonflage et le dégonflage d'au moins l'organe d'occlusion artériel.

De plus, un dispositif de mesure selon l'invention peut présenter en outre au moins l'une des caractéristiques additionnelles suivantes :
- le dispositif de gonflage et de dégonflage asservi assure également le gonflage et le dégonflage de l'organe de vidange,
- au moins l'organe d'occlusion est réalisé par une chambre à air,
- le dispositif comporte un système de mesure de pression mesurant au moins la pression appliquée par l'organe d'occlusion artériel, et de préférence également la pression appliquée par l'organe de vidange,
- le dispositif comporte un système de traitement commandé par l'électronique de commande et comprenant un étage d'acquisition qui acquiert au moins les informations délivrées par le système de mesure de pression et par le capteur, ainsi qu'une unité de traitement comportant des moyens de détermination du moment où le volume sanguin du doigt au niveau de la première zone varie sensiblement positivement et des moyens de calcul de la pression sanguine systolique correspondant à la pression d'occlusion au moment où le volume sanguin du doigt au niveau de la première zone varie sensiblement positivement,
- l'unité de traitement comporte en outre des moyens d'extraction d'une composante pulsatile représentant la circulation artérielle pulsatile dans le doigt au niveau de la première zone en fonction du temps, des moyens de détermination du moment où la composante pulsatile du signal réapparaît, et des moyens de calcul de la pression sanguine systolique correspondant à la pression d'occlusion au moment où la composante pulsatile du signal réapparaît,
- l'unité de traitement comporte des moyens d'extraction d'une composante non pulsatile représentant le remplissage du doigt résultant de la circulation artérielle pulsatile, de la microcirculation et/ou de la circulation artérielle non pulsatile en fonction du temps, des moyens de détermination du moment où la composante non pulsatile varie sensiblement positivement, et des moyens de calcul de la pression sanguine systolique correspondant à la pression d'occlusion au moment où la composante non pulsatile varie sensiblement positivement,
- l'unité de traitement comporte des moyens de comparaison entre la valeur de la pression d'occlusion et la valeur de pression sanguine systolique minimale mesurable par le dispositif, par exemple 10 mmHg,
- l'électronique de commande pilote le dispositif de gonflage et de dégonflage pour appliquer la pression de vidange antérieurement à l'application de la pression d'occlusion et pour relâcher la pression de vidange postérieurement à l'application de la pression d'occlusion et antérieurement au relâchement contrôlé de la pression d'occlusion,
- l'électronique de commande pilote l'étage d'acquisition pour acquérir pendant le relâchement contrôlé de la pression d'occlusion au moins les informations délivrées par le système de mesure de pression et par le capteur,
- l'électronique de commande pilote l'unité de traitement pour calculer la pression systolique pendant le relâchement contrôlé de la pression d'occlusion et au moment de la détection du retour sanguin dans le doigt.

Diverses autres caractéristiques ressortent de la description faite ci-dessous en référence aux dessins annexés qui montrent, à titre d'exemples non limitatifs, des formes de réalisation de l'objet de l'invention.
La **Figure 1** représente de façon schématique un dispositif selon l'invention.
La **Figure 2** représente de façon schématique un exemple de réalisation du dispositif de gonflage et de dégonflage asservi.
La **Figure 3** est un logigramme du procédé selon l'invention.
La **Figure 4A** est une courbe donnant la pression sanguine ainsi que la pression dans l'organe d'occlusion pendant le dégonflage en fonction du temps.
La **Figure 4B** est une courbe représentant le volume sanguin dans le doigt pendant le dégonflage en fonction du temps.
La **Figure 4C** est une courbe représentant la composante pulsatile pendant le dégonflage en fonction du temps.
La **Figure 4D** est une courbe représentant la composante non pulsatile pendant le dégonflage en fonction du temps.

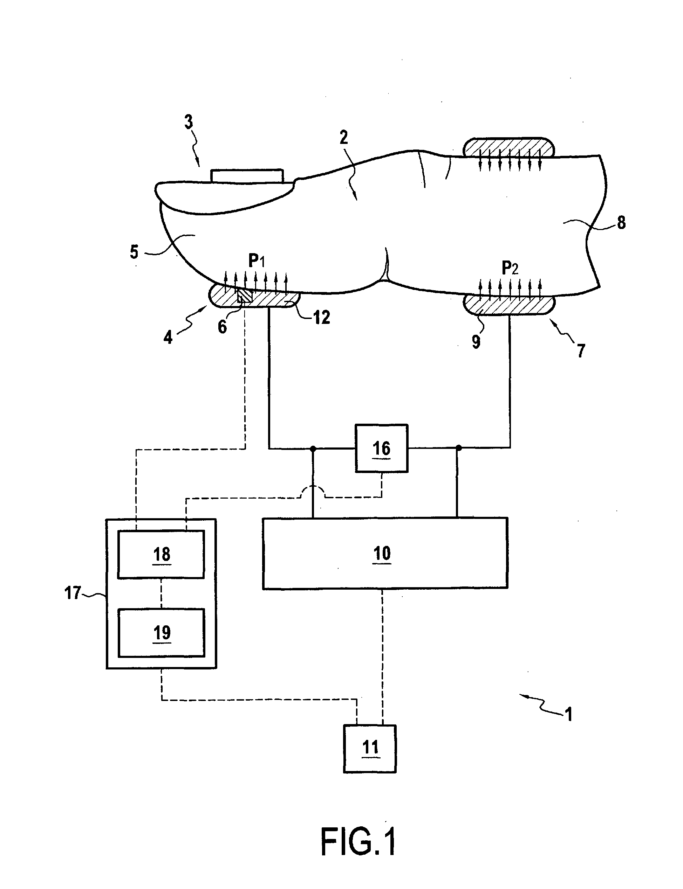

La **Figure 1** représente un exemple de réalisation d'un dispositif de mesure **1** non invasif de la pression sanguine systolique d'un patient. Le dispositif **1** est destiné à être installé sur un doigt **2** présentant une partie distale **3.**

Le dispositif de mesure **1** peut être éventuellement installé sur un doigt de la main ou avantageusement sur un doigt du pied du patient. Dans la suite de la présente description, le terme « doigt » se rapporte donc indifféremment à un doigt de la main ou à un orteil.

Le dispositif **1** comprend un premier élément **4** positionnable au niveau d'une première zone **5** du doigt **2** située à proximité de la partie distale **3.** Le premier élément **4** comporte un capteur **6** apte à mesurer le volume sanguin du doigt **2** au niveau de la première zone **5**. Le capteur **6** peut être, par exemple, du type photopléthysmographique, Doppler à ultrasons ou encore Doppler LASER.

Le dispositif **1** comprend également un deuxième élément **7** positionnable au niveau d'une deuxième zone **8** du doigt **2,** située en amont de la première zone **5.** Le deuxième élément **7** comporte un organe d'occlusion artériel **9** capable d'appliquer et/ou de maintenir une pression d'occlusion **P2,** variable et astringente, au niveau de la deuxième zone **8** du doigt **2.** Dans sa valeur maximale, la pression d'occlusion **P2** est supérieure à la pression sanguine systolique **Psyst** du patient de manière à pouvoir empêcher la circulation sanguine dans la partie du doigt **2** située en aval de la deuxième zone **8.**

L'organe d'occlusion **9** peut être réalisé par tout moyen approprié, par exemple par un dispositif pneumatique et de manière préférentielle sous forme d'une chambre à air entourant le doigt **2.** De manière avantageuse, la chambre à air permet d'obtenir une répartition uniforme de la pression d'occlusion **P2** et sa réalisation sous forme d'un bracelet lui permet d'être maintenue autour du doigt **2.**

Le gonflage et le dégonflage de l'organe d'occlusion artériel **9** sont réalisés au moyen d'un dispositif de gonflage et de dégonflage asservi **10** commandé par une électronique de commande **11.**

Conformément à l'invention, le premier élément **4** comprend également un organe de vidange **12** capable d'appliquer, de maintenir et/ou de transmettre une pression de vidange **P1** au niveau de la première zone **5** du doigt **2.** La pression de vidange **P1** est supérieure à la pression sanguine systolique **Psyst** du patient de manière à pouvoir vidanger sensiblement la partie distale **3** du sang qu'elle contient.

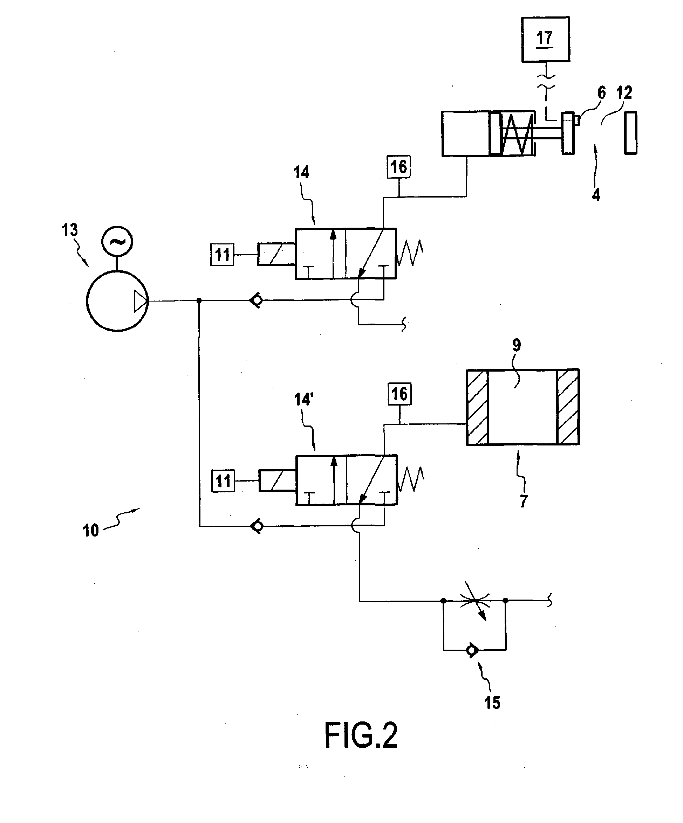

L'organe de vidange **12** peut être réalisé par tout moyen approprié et de préférence par un dispositif pneumatique, comme une chambre à air ou comme illustré sur la **Figure 2** par un vérin dont le piston est mobile en translation jusqu'à une butée. Dans le cas d'un dispositif pneumatique, les gonflage et dégonflage de l'organe de vidange **12** sont préférentiellement réalisés par le dispositif de gonflage et de dégonflage asservi **10.** De manière avantageuse, l'organe de vidange **12** est réalisé de manière à être maintenu sur le doigt **2,** par exemple sous la forme d'un bracelet ou d'une pince.

Dans une variante non représentée de l'invention, la pression de vidange **P1** est appliquée et relâchée manuellement au niveau de la première zone **5.**

La **Figure 2** représente schématiquement un exemple de réalisation d'un dispositif de gonflage et de dégonflage asservi **10.** Dans cet exemple, le dispositif **10** est apte à assurer simultanément le gonflage et le dégonflage de l'organe d'occlusion **9** et de l'organe de vidange **12.**

Le dispositif de gonflage **10** se compose d'un moyen d'alimentation pneumatique **13,** par exemple une pompe, qui alimente deux distributeurs pneumatiques **14, 14'** pilotés par l'électronique de commande **11** et reliés respectivement à l'organe d'occlusion **9** et à l'organe de vidange **12.** Dans sa position d'échappement, le distributeur **14'** relie l'organe d'occlusion **9** à un moyen de réduction du débit **15,** par exemple et comme dans l'exemple illustré un réducteur de débit.

Conformément à l'exemple de réalisation illustré, le dispositif de mesure **1** comporte en outre un système de mesure de pression **16** adapté pour mesurer au moins la pression d'occlusion **P2** appliquée par l'organe d'occlusion artériel **9.** Dans cet exemple, le système de mesure de pression **16** est également adapté pour mesurer la pression de vidange **P1** appliquée par l'organe de vidange **12.**

Le dispositif de mesure **1** comporte en outre un système de traitement **17** commandé par l'électronique de commande **11.** Le système de traitement **17** comprend un étage d'acquisition **18** qui acquiert au moins la valeur du volume sanguin **Vs** dans le doigt **2** au cours du temps, délivrée par le capteur **6,** ainsi que la valeur de la pression d'occlusion **P2** au cours du temps, délivrée par le système de mesure de pression **16.** Le système de traitement **17** comprend également une unité de traitement **19** qui calcule la pression sanguine systolique **Psyst.**

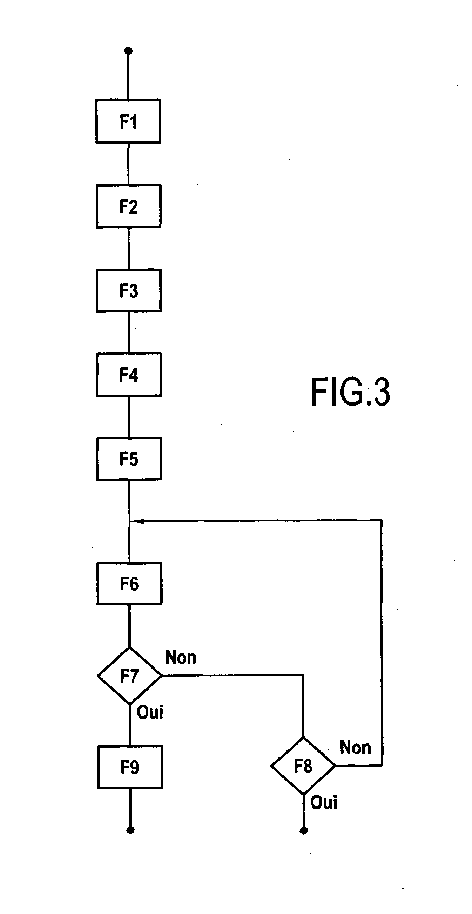

Le dispositif de mesure **1** est utilisé selon le procédé de l'invention, dont le logigramme est représenté **Figure 3****,** pour permettre la mesure de la pression systolique **Psyst** d'un patient.

Dans la première phase **F1** du procédé, la pression de vidange P1 est appliquée et maintenue, par exemple au moyen de l'organe de vidange **12,** au niveau de la première zone **5** situé à proximité de la partie distale **3** du doigt **2.** La pression de vidange **P1** est supérieure à la pression sanguine systolique **Psyst** de manière à vidanger sensiblement le doigt **2** du sang qu'il contient.

Dans la deuxième phase **F2,** la pression d'occlusion **P2** est ensuite appliquée et maintenue, par exemple au moyen de l'organe d'occlusion **9,** au niveau de la deuxième zone **8** du doigt **2** situé en amont de la première zone **5.** A cette étape, la pression d'occlusion **P2** est de préférence supérieure à la pression sanguine systolique **Psyst** de manière à empêcher un retour sanguin éventuel dans la partie distale **3.**

Dans la troisième phase **F3** du procédé, la pression de vidange **P1** est relâchée. Comme la pression d'occlusion **P2** est supérieure à la pression sanguine systolique **Psyst,** le volume sanguin **Vs** dans la partie distale **3** reste sensiblement nul.

Dans un mode préféré de réalisation, le procédé comporte une quatrième phase **F4** d'attente, d'une durée comprise entre 0 et 5s et préférentiellement de l'ordre de 3s. De manière avantageuse, cette attente permet de garantir la stabilisation des pressions **P1** et **P2** avant la mesure.

Dans la cinquième phase **F5**, la pression d'occlusion **P2** est relâchée de manière contrôlée, par exemple grâce au moyen de réduction de débit **15.** La pression d'occlusion **P2** chute alors continuellement dans les étapes suivantes du procédé.

Le dégonflage est préférentiellement linéaire ou différentiel, c'est-à-dire de la forme **P2**(t)= *P*₀·*e*^{-*t*/*T*}. De manière avantageuse, un dégonflage différentiel permet d'obtenir une erreur de mesure sensiblement constante quelle que soit la pression d'occlusion **P2** au cours du dégonflage.

Selon un mode préféré de réalisation, le temps nécessaire au relâchement de la pression d'occlusion **P2** est ajustable de manière à se dérouler sur un nombre fixe de cycles cardiaques. Avantageusement, cette caractéristique permet d'obtenir une erreur de mesure sensiblement constante quel que soit le rythme cardiaque du patient.

Dans un mode préféré de réalisation, les pressions de vidange **P1** et d'occlusion **P2** sont appliquées et relâchées automatiquement, par exemple au moyen du dispositif de gonflage et de dégonflage **10.** Bien entendu, il est également possible d'appliquer et de relâcher manuellement la pression de vidange **P1** et d'appliquer et de relâcher automatiquement la pression d'occlusion **P2.**

Dans la sixième phase **F6** du procédé, au moins la valeur du volume sanguin au niveau de la première zone **5** et au moins la valeur de la pression d'occlusion **P2** à l'instant *t* sont acquises, par exemple au moyen de l'étage d'acquisition **18** du système de traitement **17.**

La septième phase **F7** consiste en la détection de la présence éventuelle d'un retour sanguin au niveau de la première zone **5.** Le retour sanguin est caractérisé par le remplissage en sang du doigt **2,** et donc par une variation sensiblement positive du volume sanguin **Vs** du doigt **2** au niveau de la première zone **5.**

S'il n'y a pas de retour sanguin, la huitième phase **F8** consiste en la comparaison de la valeur de la pression d'occlusion **P2** à l'instant t avec la valeur minimale de pression sanguine systolique **Psyst** mesurable par le procédé, par exemple 10 mmHg. Si la valeur de la pression d'occlusion **P2** est inférieure ou égale à cette valeur minimale, la mesure se termine. Si la valeur de la pression d'occlusion **P2** est supérieure à cette valeur minimale, la sixième phase **F6** ainsi que les suivantes sont répétées.

S'il y a retour sanguin, la valeur de la pression sanguine systolique **Psyst** est calculée dans la neuvième phase **F9** du procédé en fonction des informations acquises.

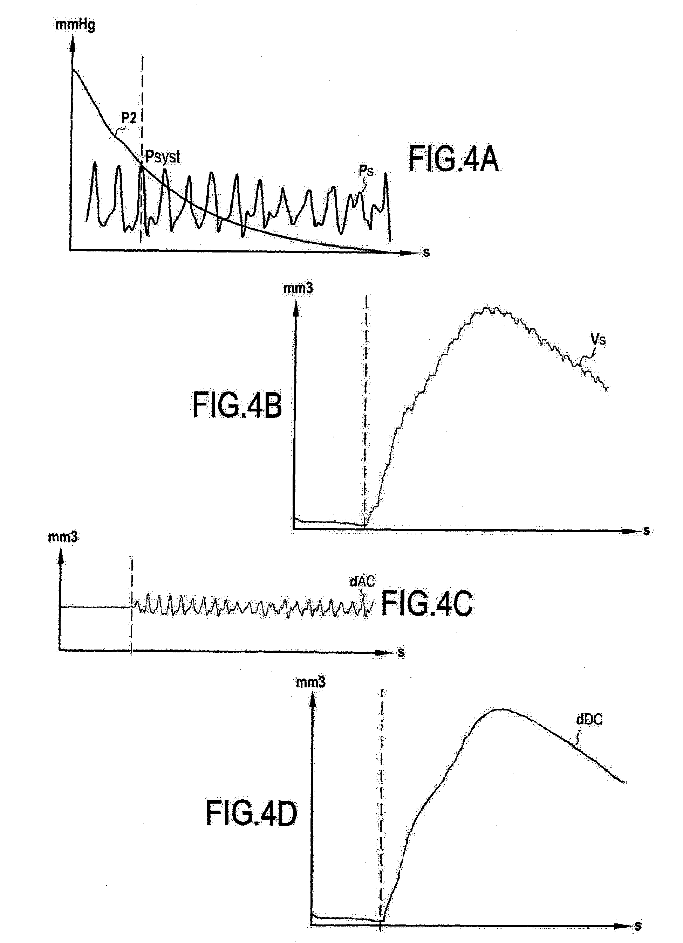

Les **Figures 4A** à **4D** illustrent le mode de calcul de la pression sanguine systolique **Psyst.**

La **Figure 4A** est une courbe donnant la pression sanguine artérielle **Ps** d'un patient, exprimée en mmHg, et la valeur de la pression d'occlusion **P2,** exprimée en mmHg, en fonction du temps.

La **Figure 4B** donne la valeur du volume sanguin **Vs** dans le doigt **2** au niveau de la première zone **5**, exprimé en mm³, en fonction du temps.

Tant que la pression d'occlusion **P2** est sensiblement supérieure à la pression sanguine **Ps,** le volume sanguin **Vs** est sensiblement nul du fait de la vidange du doigt **2** opérée préalablement à la mesure. Lorsque la pression d'occlusion **P2** devient sensiblement égale à la pression sanguine **Ps,** le retour sanguin apparaît et le volume sanguin **Vs** varie sensiblement positivement. Le début du remplissage se produit nécessairement sur une impulsion systolique, où la valeur de pression **Ps** est la plus élevée. De ce fait, la pression sanguine systolique **Psyst** du patient correspond à la pression d'occlusion **P2** au moment du retour sanguin du doigt **2.**

Ainsi, le remplissage du doigt **2** est dû aux effets combinés de la circulation artérielle pulsatile, de la micro-circulation et de la circulation artérielle non pulsatile. Cette combinaison augmente avantageusement la dynamique du remplissage, plus particulièrement au début du remplissage, et permet de disposer d'une précision de mesure optimale même dans le cas où le flux sanguin du patient est peu pulsatile, c'est-à-dire dans le cas de pressions sanguines faibles.

Dans une variante du procédé, la variation du volume sanguin **Vs** au cours du temps est traitée, par exemple par filtrage, pour en extraire une composante pulsative **dAC** et/ou une composante non pulsatile **dDC.**

La composante pulsatile **dAC,** représentée sur la **Figure 4C** et s'exprimant en mm³, représente le débit de remplissage du doigt 2 dû à la circulation artérielle pulsatile en fonction du temps. La composante pulsatile **dAC** est à moyenne nulle et présente des variations croissantes et décroissantes.

La composante non pulsatile **dDC,** représentée sur la **Figure 4D** et s'exprimant en mm³, représente le remplissage du doigt 2 résultant de la circulation artérielle pulsatile, de la microcirculation et/ou de la circulation artérielle non pulsatile en fonction du temps.

La sommation de la composante non pulsatile **dDC** et de l'intégrale de la composante pulsatile **dAC** permet sensiblement d'obtenir la courbe du volume sanguin **Vs** au cours du temps.

Tant que la pression d'occlusion **P2** est sensiblement supérieure à la pression sanguine **Ps,** les composantes pulsatiles **dAC** et non pulsatiles **dDC** sont sensiblement nulles puisqu'il n'y a aucun flux sanguin dans le doigt **2.** Lorsque la pression d'occlusion **P2** devient sensiblement égale à la pression sanguine **Ps,** le retour sanguin réapparaît ainsi que la composante pulsatile **dAC,** tandis que la composante non pulsatile **dDC** varie sensiblement positivement.

De ce fait, la valeur de la pression sanguine systolique **Psyst** peut être calculée à partir de la composante pulsatile **dAC** par détermination de la pression d'occlusion **P2** au moment où la composante pulsatile **dAC** du signal réapparaît. Cela permet avantageusement d'augmenter la précision de la mesure en l'échantillonnant par le cycle cardiaque.

Par ailleurs, la valeur de la pression sanguine systolique **Psyst** peut également être calculée à partir de la composante non pulsatile **dDC** par détermination de la pression d'occlusion **P2** au moment où la composante non pulsatile **dDC** varie sensiblement positivement. Ce mode de détermination de la pression sanguine systolique **Psyst** demande avantageusement une dynamique de traitement plus faible que la méthode basée sur l'étude de la variation du volume sanguin au cours du temps.

De manière avantageuse, le fait de disposer d'une pluralité de méthodes pour calculer la pression systolique **Psyst** permet de disposer d'un appareil polyvalent capable de s'adapter à une large palette de situations.

Dans l'exemple illustré de réalisation du dispositif de mesure **1,** la neuvième phase **F9** de calcul de la pression sanguine systolique **Psyst** est réalisée au moyen de l'unité de traitement **19.**

A cet effet, l'unité de traitement **19** comporte des moyens de détermination du moment où le volume sanguin **Vs** du doigt **2** au niveau de la première zone 5 varie sensiblement positivement, et des moyens de calcul de la pression sanguine systolique **Psyst** correspondant à la pression d'occlusion **P2** au moment où le volume sanguin **Vs** du doigt **2** au niveau de la première zone **5** varie sensiblement positivement.

Dans une variante de réalisation, l'unité de traitement **19** comporte également des moyens d'extraction d'une composante pulsatile **dAC** représentant la circulation artérielle pulsatile dans le doigt **2** au niveau de la première zone **5** en fonction du temps, des moyens de détermination du moment où la composante pulsatile **dAC** du signal réapparaît et des moyens de calcul de la pression sanguine systolique **Psyst** correspondant à la pression d'occlusion **P2** au moment où la composante pulsatile **dAC** du signal réapparaît.

Dans une autre variante de réalisation, l'unité de traitement **19** comporte, pour le calcul de la pression sanguine systolique **Psyst,** des moyens d'extraction d'une composante non pulsatile **dDC** représentant le remplissage du doigt **2** résultant de la circulation artérielle pulsatile, de la micro-circulation et/ou de la circulation artérielle non pulsatile en fonction du temps, des moyens de détermination du moment où la composante non pulsatile **dDC** varie sensiblement positivement, ainsi que des moyens de calcul de la pression sanguine systolique **Psyst** correspondant à la pression d'occlusion **P2** au moment où la composante non pulsatile **dDC** varie sensiblement positivement.

Pour mettre en oeuvre le procédé selon l'invention, l'unité de traitement **19** comporte par ailleurs des moyens de comparaison entre la valeur de la pression d'occlusion **P2** et la valeur de pression sanguine systolique **Psyst** minimale mesurable par le dispositif **1,** par exemple 10 mmHg.

Dans le but de permettre la mise en oeuvre, par le dispositif **1,** du procédé selon l'invention, l'électronique de commande **11** pilote le dispositif de gonflage et de dégonflage **10** d'une part pour appliquer la pression de vidange **P1** antérieurement à la pression d'occlusion **P2,** et d'autre part pour relâcher la pression de vidange **P1** postérieurement à l'application de la pression d'occlusion **P2** et antérieurement au relâchement contrôlé de la pression d'occlusion **P2.**

L'électronique de commande **11** pilote également l'étage d'acquisition **18** pour acquérir pendant le relâchement contrôlé de la pression d'occlusion **P2 au** moins les informations délivrées par le système de mesure de pression **16** et par le capteur **6.**

Par ailleurs, l'électronique de commande **11** pilote l'unité de traitement **19** pour calculer la pression systolique **Psyst** pendant le relâchement contrôlé de la pression d'occlusion **P2** et au moment de la détection du retour sanguin dans le doigt **2.**

## Revendications

1. Procédé non invasif de mesure de la pression sanguine systolique (**Psyst**) d'un doigt (**2**) et notamment de l'orteil comportant une partie distale (**3**), ce procédé comprenant les étapes suivantes :
- appliquer et maintenir une pression de vidange (**P1**) astringente sur une première zone (**5**) située à proximité de la partie distale (**3**) du doigt (**2**),
- appliquer et maintenir une pression d'occlusion (**P2**) astringente et supérieure à la pression sanguine systolique (**Psyst**) du doigt (**2**) sur une deuxième zone (**8**) du doigt (**2**) située en amont de la première zone (**5**),
- relâcher la pression de vidange (**P1**),
- relâcher la pression d'occlusion (**P2**) de manière contrôlée, et dans le même temps acquérir au moins la pression d'occlusion (**P2**) et au moins le volume sanguin (**Vs**) du doigt (**2**) au niveau de la première zone (**5**) en fonction du temps,
- calculer la pression sanguine systolique (**Psyst**) correspondant à la pression d'occlusion (**P2**) au moment du retour sanguin du doigt (**2**) révélé par une variation sensiblement positive du volume sanguin (**Vs**) du doigt (**2**) au niveau de la première zone (**5**).

2. Procédé de mesure de la pression sanguine selon la revendication 1, **caractérisé en ce que** la pression de vidange (**P1**) est supérieure à la pression sanguine systolique (**Psyst**) du doigt (**2**).

3. Procédé de mesure de la pression sanguine selon la revendication 1 ou 2, **caractérisé en ce que** les pressions de vidange (**P1**) et d'occlusion (**P2**) sont appliquées et relâchées automatiquement.

4. Procédé de mesure de la pression sanguine selon l'une des revendications 1 à 3, **caractérisé en ce que** la pression de vidange (**P1**) est appliquée et relâchée manuellement tandis que la pression d'occlusion (**P2**) est appliquée et relâchée automatiquement.

5. Procédé de mesure de la pression sanguine selon l'une des revendications 1 à 4, **caractérisé en ce qu'**il comporte une phase d'attente (**F4**) d'une durée comprise entre 0 et 5s et préférentiellement de l'ordre de 3s, située après le relâchement de la pression de vidange (**P1**).

6. Procédé de mesure de la pression sanguine selon l'une des revendications 1 à 5, caractérisé en que l'étape de calcul de la pression sanguine systolique (**Psyst**) consiste à :
- extraire une composante pulsatile (**dAC**) représentant la circulation artérielle pulsatile dans le doigt (**2**), à partir de la variation du volume sanguin (**Vs**) du doigt (**2**) au niveau de la première zone (**5**) en fonction du temps,
- calculer la pression sanguine systolique (**Psyst**) par détermination de la pression d'occlusion (**P2**) au moment où la composante pulsatile (**dAC**) du signal réapparaît.

7. Procédé de mesure de la pression sanguine selon l'une des revendications 1 à 5, caractérisé en que l'étape de calcul de la pression sanguine systolique (**Psyst**) consiste à :
- extraire une composante non pulsatile (**dDC**) représentant le remplissage du doigt (**2**) résultant de la circulation artérielle pulsatile, de la microcirculation et/ou de la circulation artérielle non pulsatile, à partir de la variation du volume sanguin (**Vs**) du doigt (**2**) au niveau de la première zone (**5**) en fonction du temps,
- calculer la pression sanguine systolique (**Psyst**) en déterminant la pression d'occlusion (**P2**) au moment où la composante non pulsatile (**dDC**) varie sensiblement positivement.

8. Procédé de mesure de la pression sanguine selon l'une des revendications 1 à 7, **caractérisé en ce qu'**il consiste à ajuster le temps nécessaire au relâchement de la pression d'occlusion (**P2**) pour qu'il se déroule sur un nombre de cycles cardiaques fixe.

9. Dispositif de mesure (**1**) non invasif de la pression sanguine systolique (**Psyst**) d'un doigt (**2**) muni d'une partie distale (**3**), le dispositif comportant :
- un premier élément (**4**) comprenant un capteur (**6**), apte à mesurer le volume sanguin (**Vs**) du doigt (**2**) au niveau d'une première zone (**5**) située à proximité de la partie distale (**3**) du doigt (**2**), et un organe de vidange (**12**), apte à appliquer ou à transmettre une pression de vidange (**P1**) sur la première zone (**5**) du doigt (**2**),
- un deuxième élément (**7**) comprenant un organe d'occlusion artériel (**9**), apte à appliquer et/ou à maintenir une pression d'occlusion (**P2**) sur une deuxième zone (**8**) du doigt (**2**) situé en amont de la première zone (**5**), et
- un dispositif de gonflage et de dégonflage (**10**) asservi commandé par une électronique de commande (**11**) et assurant le gonflage et le dégonflage d'au moins l'organe d'occlusion artériel (**9**),
- un système de mesure de pression (**16**) mesurant au moins la pression appliquée par l'organe d'occlusion artériel (**9**), l'électronique de commande (**11**) pilotant le dispositif de gonflage et de dégonflage (**10**) afin que l'organe d'occlusion artériel (**9**) applique et maintienne une pression d'occlusion (**P2**) supérieure à la pression sanguine systolique (**Psyst**) du doigt (**2**), antérieurement à un relâchement contrôlé de la pression d'occlusion (**P2**),
- et un système de traitement (**17**) commandé par l'électronique de commande (**11**) et comprenant :
- un étage d'acquisition (**18**) qui acquiert au moins pendant le relâchement contrôlé de la pression d'occlusion (**P2**) au moins les informations délivrées par le système de mesure de pression (**16**) et par le capteur (**6**),
- une unité de traitement (**19**) comportant :
a. des moyens de détermination du moment où le volume sanguin (**Vs**) du doigt (**2**) au niveau de la première zone (**5**) varie sensiblement positivement,
b. des moyens de calcul de la pression sanguine systolique (**Psyst**) correspondant à la pression d'occlusion (**P2**) au moment où le volume sanguin (**Vs**) du doigt (**2**) au niveau de la première zone (**5**) varie sensiblement positivement.

10. Dispositif de mesure (**1**) selon la revendication 9, **caractérisé en ce que** le dispositif de gonflage et de dégonflage (**10**) assure également le gonflage et le dégonflage de l'organe de vidange (**12**).

11. Dispositif de mesure (**1**) selon la revendication 9 ou 10, **caractérisé en ce qu'**au moins l'organe d'occlusion (**9**) est réalisé par une chambre à air.

12. Dispositif de mesure (**1**) selon la revendication 9 et **caractérisé en ce que** l'unité de traitement (**19**) comporte en outre :
- des moyens d'extraction d'une composante pulsatile (**dAC**) représentant la circulation artérielle pulsatile dans le doigt (**2**) au niveau de la première zone (**5**) en fonction du temps,
- des moyens de détermination du moment où la composante pulsatile (**dAC**) du signal réapparaît,
- des moyens de calcul de la pression sanguine systolique (**Psyst**) correspondant à la pression d'occlusion (**P2**) au moment où la composante pulsatile (**dAC**) du signal réapparaît.

13. Dispositif de mesure (**1**) selon la revendication 9 et caractérisé ce que l'unité de traitement (**19**) comporte en outre :
- des moyens d'extraction d'une composante non pulsatile (**dDC**) représentant le remplissage du doigt (**2**) résultant de la circulation artérielle pulsatile, de la microcirculation et/ou de la circulation artérielle non pulsatile en fonction du temps,
- des moyens de détermination du moment où la composante non pulsatile (**dDC**) varie sensiblement positivement,
- des moyens de calcul de la pression sanguine systolique (**Psyst**) correspondant à la pression d'occlusion (**P2**) au moment où la composante non pulsatile (**dDC**) varie sensiblement positivement.

14. Dispositif de mesure (**1**) selon l'une des revendications 9 à 13, caractérisé ce que l'unité de traitement (**19**) comporte des moyens de comparaison entre la valeur de la pression d'occlusion (**P2**) et la valeur de pression sanguine systolique (**Psyst**) minimale mesurable par le dispositif (**1**), par exemple 10 mmHg.

15. Dispositif de mesure (**1**) selon l'une des revendications 9 à 14, caractérisé ce que l'électronique de commande (**11**) pilote :
- le dispositif de gonflage et de dégonflage (**10**) pour appliquer la pression de vidange (**P1**) antérieurement à l'application de la pression d'occlusion (**P2**),
- le dispositif de gonflage et de dégonflage (**10**) pour relâcher la pression de vidange (**P1**) postérieurement à l'application de la pression d'occlusion (**P2**) et antérieurement au relâchement contrôlé de la pression d'occlusion (**P2**),
- l'unité de traitement (**19**) pour calculer la pression systolique (**Psyst**) pendant le relâchement contrôlé de la pression d'occlusion (**P2**) et au moment de la détection du retour sanguin dans le doigt (**2**).

## Patentansprüche

1. Nicht-invasives Verfahren zur Messung des systolischen Blutdrucks (Psyst) eines Fingers (2) und insbesondere des Zehs, der einen distalen Teil (3) umfasst, wobei dieses Verfahren die folgenden Schritte umfasst:
- Anlegen und Halten eines adstringierenden Entleerungsdrucks (P1) an einen/einem ersten Bereich (5), der in der Nähe des distalen Teils (3) des Fingers (2) gelegen ist,
- Anlegen und Halten eines adstringierenden und oberhalb des systolischen Blutdrucks (Psyst) des Fingers (2) gelegenen Okklusionsdrucks (P2) an einen/einem zweiten Bereich (8) des Fingers (2), der vor dem ersten Bereich (5) gelegen ist,
- Vermindern des Entleerungsdrucks (P1),
- kontrolliertes Vermindern des Okklusionsdrucks (P2) und gleichzeitig Erfassen wenigstens des Okklusionsdrucks (P2) und wenigstens des Blutvolumens (Vs) des Fingers (2) in Höhe des ersten Bereichs (5) in Abhängigkeit von der Zeit,
- Berechnen des systolischen Blutdrucks (Psyst), der dem Okklusionsdruck (P2) zum Zeitpunkt der Blutrückkehr des Fingers (2) entspricht, die durch eine im Wesentlichen positive Änderung des Blutvolumens (Vs) des Fingers (2) in Höhe des ersten Bereichs (5) festgestellt wird.

2. Verfahren zur Messung des Blutdrucks nach Anspruch 1, **dadurch gekennzeichnet, dass** der Entleerungsdruck (P1) größer als der systolische Blutdruck (Psyst) des Fingers (2) ist.

3. Verfahren zur Messung des Blutdrucks nach Anspruch 1 oder 2, **dadurch gekennzeichnet, dass** der Entleerungs- (P1) und der Okklusionsdruck (P2) automatisch angelegt und vermindert werden.

4. Verfahren zur Messung des Blutdrucks nach einem der Ansprüche 1 bis 3, **dadurch gekennzeichnet, dass** der Entleerungsdruck (P1) manuell angelegt und vermindert wird, während der Okklusionsdruck (P2) automatisch angelegt und vermindert wird.

5. Verfahren zur Messung des Blutdrucks nach einem der Ansprüche 1 bis 4, **dadurch gekennzeichnet, dass** es eine Wartephase (F4) mit einer Dauer zwischen 0 und 5 s und vorzugsweise in der Größenordnung von 3 s umfasst, die nach dem Vermindern des Entleerungsdrucks (P1) liegt.

6. Verfahren zur Messung des Blutdrucks nach einem der Ansprüche 1 bis 5, **dadurch gekennzeichnet, dass** der Schritt zur Berechnung des systolischen Blutdrucks (Psyst) darin besteht:
- eine pulsatile Komponente (dAC), welche den pulsatilen arteriellen Kreislauf in dem Finger (2) darstellt, aus der Änderung des Blutvolumens (Vs) des Fingers (2) in Höhe des ersten Bereichs (5) in Abhängigkeit von der Zeit zu gewinnen,
- den systolischen Blutdruck (Psyst) durch Bestimmen des Okklusionsdrucks (P2) zu dem Zeitpunkt, wo die pulsatile Komponente (dAC) des Signals wieder auftritt, zu berechnen.

7. Verfahren zur Messung des Blutdrucks nach einem der Ansprüche 1 bis 5, **dadurch gekennzeichnet, dass** der Schritt zur Berechnung des systolischen Blutdrucks (Psyst) darin besteht:
- eine nicht pulsatile Komponente (dDC), welche das Füllen des Fingers (2), das aus dem pulsatilen arteriellen Kreislauf, der Mikrozirkulation und/oder dem nicht pulsatilen arteriellen Kreislauf resultiert, darstellt, aus der Änderung des Blutvolumens (Vs) des Fingers (2) in Höhe des ersten Bereichs (5) in Abhängigkeit von der Zeit zu gewinnen,
- den systolischen Blutdruck (Psyst) dadurch zu berechnen, dass der Okklusionsdruck (P2) zu dem Zeitpunkt bestimmt wird, wo die nicht pulsatile Komponente (dDC) sich im Wesentlichen positiv ändert.

8. Verfahren zur Messung des Blutdrucks nach einem der Ansprüche 1 bis 7, **dadurch gekennzeichnet, dass** es darin besteht, die Zeit, die für das Vermindern des Okklusionsdrucks (P2) erforderlich ist, einzustellen, damit es über eine feste Anzahl von Herzzyklen abläuft.

9. Nicht-invasive Vorrichtung zur Messung (1) des systolischen Blutdrucks (Psyst) eines Fingers (2), der mit einem distalen Teil (3) versehen ist, wobei die Vorrichtung umfasst:
- ein erstes Element (4) mit einem Sensor (6), das geeignet ist, das Blutvolumen (Vs) des Fingers (2) in Höhe eines ersten Bereichs (5), welcher in der Nähe des distalen Teils (3) des Fingers (2) gelegen ist, zu messen, sowie ein Entleerungsorgan (12), das geeignet ist, einen Entleerungsdruck (P1) an/auf den ersten Bereich (5) des Fingers (2) anzulegen oder zu übertragen,
- ein zweites Element (7) mit einem arteriellen Okklusionsorgan (9), das geeignet ist, einen Okklusionsdruck (P2) an einen/einem zweiten Bereich (8) des Fingers (2), welcher vor dem ersten Bereich (5) gelegen ist, anzulegen und/oder zu halten, und
- eine gesteuerte Aufblas- und Ablassvorrichtung (10), die durch eine Steuerelektronik (11) gesteuert wird und das Aufblasen und das Entleeren wenigstens des arteriellen Okklusionsorgans (9) sicherstellt,
- ein Druckmesssystem (16), das wenigstens den durch das arterielle Okklusionsorgan (9) angelegten Druck misst, wobei die Steuerelektronik (11) die Aufblas- und Ablassvorrichtung (10) steuert, damit das arterielle Okklusionsorgan (9) einen Okklusionsdruck (P2) oberhalb des systolischen Blutdrucks (Psyst) des Fingers (2) vor einem kontrollierten Vermindern des Okklusionsdrucks (P2) anlegt und hält,
- und ein Verarbeitungssystem (17), das durch die Steuerelektronik (11) gesteuert wird und umfasst:
- eine Erfassungsstufe (18), die wenigstens während des kontrollierten Verminderns des Okklusiflnsdrucks (P2) wenigstens die durch das Druckmesssystem (16) und durch den Sensor (6) gelieferten Informationen erfasst,
- eine Verarbeitungseinheit (19), umfassend:
a. Mittel zum Bestimmen des Zeitpunkts, zu dem das Blutvolumen (Vs) des Fingers (2) in Höhe des ersten Bereichs (5) sich im Wesentlichen positiv ändert,
b. Mittel zum Berechnen des systolischen Blutdrucks (Psyst), der dem Okklusionsdruck (P2) zu dem Zeitpunkt entspricht, wo das Blutvolumen (Vs) des Fingers (2) in Höhe des ersten Bereichs (5) sich im Wesentlichen positiv ändert.

10. Messvorrichtung (1) nach Anspruch 9, **dadurch gekennzeichnet, dass** die Aufblas-und Ablassvorrichtung (10) auch das Aufblasen und Entleeren des Entleerungsorgans (12) sicherstellt.

11. Messvorrichtung (1) nach Anspruch 9 oder 10, **dadurch gekennzeichnet, dass** wenigstens das Okklusionsorgan (9) durch eine Luftkammer realisiert ist.

12. Messvorrichtung (1) nach Anspruch 9 und **dadurch gekennzeichnet, dass** die Verarbeitungseinheit (19) ferner umfasst:
- Mittel zum Gewinnen einer pulsatilen Komponente (dAC), welche den pulsatilen arteriellen Kreislauf in dem Finger (2) in Höhe des ersten Bereichs (5) in Abhängigkeit von der Zeit darstellt,
- Mittel zum Bestimmen des Zeitpunkts, wo die pulsatile Komponente (dAC) des Signals wieder auftritt,
- Mittel zum Berechnen des systolischen Blutdrucks (Psyst), der dem Okklusionsdruck (P2) zu dem Zeitpunkt entspricht, wo die pulsatile Komponente (dAC) des Signals wieder auftritt.

13. Messvorrichtung (1) nach Anspruch 9 und **dadurch gekennzeichnet, dass** die Verarbeitungseinheit (19) ferner umfasst:
- Mittel zum Gewinnen einer nicht pulsatilen Komponente (dDC), welche das Füllen des Fingers (2), das aus dem pulsatilen arteriellen Kreislauf, der Mikrozirkulation und/oder dem nicht pulsatilen arteriellen Kreislauf resultiert, in Abhängigkeit von der Zeit darstellt,
- Mittel zum Bestimmen des Zeitpunkts, wo die nicht pulsatile Komponente (dDC) sich im Wesentlichen positiv ändert,
- Mittel zum Berechnen des systolischen Blutdrucks (Psyst), der dem Okklusionsdruck (P2) zu dem Zeitpunkt entspricht, wo die nicht pulsatile Komponente (dDC) sich im Wesentlichen positiv ändert.

14. Messvorrichtung (1) nach einem der Ansprüche 9 bis 13, **dadurch gekennzeichnet, dass** die Verarbeitungseinheit (19) Mittel zum Vergleichen des Wertes des Okklusionsdrucks (P2) mit dem Wert des durch die Vorrichtung (1) messbaren minimalen systolischen Blutdrucks (Psyst), beispielsweise 10 mmHg, umfasst.

15. Messvorrichtung (1) nach einem der Ansprüche 9 bis 14, **dadurch gekennzeichnet, dass** die Steuerelektronik (11) steuert:
- die Aufblas- und Ablassvorrichtung (10), um den Entleerungsdruck (P1) vor dem Anlegen des Okklusionsdrucks (P2) anzulegen,
- die Aufblas- und Ablassvorrichtung (10), um den Entleerungsdruck (P1) nach dem Anlegen des Okklusionsdrucks (P2) und vor dem kontrollierten Vermindern des Okklusionsdrucks (P2) zu vermindern,
- die Verarbeitungseinheit (19), um den systolischen Druck (Psyst) während des kontrollierten Verminderns des Okklusionsdrucks (P2) und zum Zeitpunkt der Erfassung der Blutrückkehr in den Finger (2) zu berechnen.

## Claims

1. Non-invasive method for measuring the systolic blood pressure (**Psyst**) of a finger (**2**) and in particular of a toe having a distal part (**3**), this method comprising the following steps:
- applying and maintaining an astringent draining pressure (**P1**) on a first region (**5**) located in the vicinity of the distal part (**3**) of the finger/toe (**2**),
- applying and maintaining an astringent occluding pressure (**P2**) greater than the systolic blood pressure (**Psyst**) of the finger/toe (**2**) on a second region of the finger/toe (**2**) located upstream of the first region (**5**),
- releasing the draining pressure (**P1**),
- controllably releasing the occluding pressure (**P2**) and at the same time acquiring at least the occluding pressure (**P2**) and at least the blood volume (**Vs**) of the finger/toe (**2**) at the first region (**5**) as a function of time,
- calculating the systolic blood pressure (**P_{syst}**) corresponding to the occluding pressure (**P2**) at the time of blood return to the finger/toe (**2**) revealed by a substantially positive variation in the blood volume (**Vs**) of the finger/toe (**2**) at the first region (**5**).

2. The method for measuring blood pressure according to claim 1, **characterized in that** the draining pressure (**P1**) is greater than the systolic blood pressure (**P_{syst}**) of the finger/toe (**2**).

3. The method for measuring blood pressure according to claim 1 or 2, **characterized in that** the draining (**P1**) and occlusion (**P2**) pressures are applied and released automatically.

4. The method for measuring blood pressure according to any of claims 1 to 3, **characterized in that** the draining pressure (**P1**) is applied and released manually, whilst the occluding pressure (**P2**) is applied and released automatically.

5. The method for measuring blood pressure according to any of claims 1 to 4, **characterized in that** it comprises a waiting phase (**F4**) which lasts between 0 and 5s and preferably of the order of 3s, taking place after release of the draining pressure (**P1**).

6. The method for measuring blood pressure according to any of claims 1 to 5, **characterized in that** the step for calculating the systolic blood pressure (**P_{syst}**) consists of:
- extracting a pulsatile component (**dAC**) representing the pulsatile arterial flow in the finger/toe (**2**), from the variation in blood volume (**Vs**) of the finger/toe (**2**) at the first region (**5**) as a function of time,
- calculating the systolic blood pressure (**P_{syst}**) by determining the occluding pressure (**P2**) at the time of re-onset of the pulsatile component (**dAC**) of the signal.

7. The method for measuring blood pressure according to any of claims 1 to 5, **characterized in that** the step for calculating systolic blood pressure (**P_{syst}**) consists of:
- extracting a non-pulsatile component (**dDC**) representing filling of the finger/toe (**2**) resulting from pulsatile arterial flow, from microcirculation and/or from non-pulsatile arterial flow, using the variation in blood volume (**Vs**) of the finger/toe (**2**) at the first region (**5**) as a function of time,
- calculating systolic blood pressure (**P_{syst}**) by determining the occluding pressure (**P2**) at the time when the non-pulsatile component (**dDC**) varies substantially positively.

8. The method for measuring blood pressure according to any of claims 1 to 7, **characterized in that** it consists of adjusting the time needed for release of the occluding pressure (**P2**) so that it takes place over a fixed number of cardiac cycles.

9. A non-invasive device (**1**) for measuring the systolic blood pressure (**P_{syst}**) of a finger/toe (**2**) having a distal part (3), this device comprising:
- a first element (**4**) comprising a sensor (**6**) capable of measuring the blood volume (**Vs**) of a finger/toe (**2**) at a first region (**5**) located in the vicinity of the distal part (**3**) of the finger/toe (**2**), and a draining member (**12**) capable of applying or of transmitting a draining pressure (**P1**) to the first region (**5**) of the finger/toe (**2**),
- a second element (**7**) comprising an arterial occluding member (**9**) capable of applying and/or maintaining an occluding pressure (**P2**) on a second region (**8**) of the finger/toe (**2**) located upstream of the first region (**5**),
and
- a servo-controlled inflating and deflating device (**10**) controlled by control electronics (**11**) and ensuring the inflation and deflation of at least the arterial occluding member (**9**),
- a pressure measurement system (**16**) measuring at least the pressure applied by the arterial occluding member (**9**), the control electronic (**11**) driving the servo-controlled inflating and deflating device (**10**) in order that arterial occluding member (**9**), applies and maintains an occluding pressure (**P2**) greater than the systolic blood pressure (**P_{syst}**) of the finger/toe (**2**), before a controlled release of the occluding pressure (**P2**),
- and a processing system (**17**) controlled by the control electronics (**11**) and comprising:
. an acquisition stage (**18**) which acquires at least during the controlled release of the occluding pressure (**P2**) at least the data delivered by the pressure measurement system (**16**) and by the sensor (**6**),
. a processing unit (**19**) comprising:
a. means for determining the time when the blood volume (**Vs**) of the finger/toe (**2**) at the first region (**5**) varies substantially positively,
b. means for calculating systolic blood pressure (**P_{syat}**) corresponding to the occluding pressure (**P2**) at the time when the blood volume (**Vs**) in the finger/toe (**2**) at the first region (**5**) varies substantially positively.

10. The measuring device (**1**) according to claim 9, **characterized in that** the inflating and deflating device (**10**) also ensures the inflation and deflation of the draining member (**12**).

11. The measuring device (**1**) according to claim 9 or 10, **characterized in that** at least the occluding member (**9**) is formed of an air chamber.

12. The measuring device (**1**) according to claim 9 and **characterized in that** the processing unit (**19**) further comprises:
- means for extracting a pulsatile component (**dAC**) representing the pulsatile arterial flow in the finger/toe (**2**) at the first region (**5**) as a function of time,
- means for determining the time of re-onset of the pulsatile component (**dAC**) of the signal,
- means for calculating the systolic blood pressure (**P_{syst}**) corresponding to the occluding pressure (**P2**) at the time of re-onset of the pulsatile component (**dAC**) of the signal.

13. The measuring device (**1**) according to claim 9 and **characterized in that** the processing unit (**19**) further comprises:
- means for extracting a non-pulsatile component (**dDC**) representing filling of the finger/toe (**2**) resulting from pulsatile arterial flow, from microcirculation and/or from non-pulsatile arterial flow, as a function of time,
- means for determining the time when the non-pulsatile component (**dDC**) varies substantially positively,
- means for calculating the systolic blood pressure (**P_{syst}**) corresponding to the occluding pressure (**P2**) at the time when the non-pulsatile component (**dDC**) varies substantially positively.

14. The measuring device (**1**) according to any of claims 9 to 13, **characterized in that** the processing unit (**19**) comprises comparison means between the value of the occluding pressure (**P2**) and the value of the minimum systolic blood pressure (**P_{syst}**) which can be measured by the device (**1**), for example 10 mmHg.

15. The measuring device (**1**) according to any of claims 9 to 14, **characterized in that** the control electronics (**11**) drive:
- the inflating and deflating device (**10**) to apply the draining pressure (**P1**) before application of the occluding pressure (**P2**),
- the inflating and deflating device (**10**), to release the draining pressure (**P1**) after application of the occluding pressure (**P2**) and before the controlled release of the occluding pressure (**P2**),
- the processing unit (**19**) to calculate the systolic pressure (**P_{syat}**) during the controlled release of the occluding pressure (**P2**) and at the time of detection of blood return into the finger/toe (**2**).
